# EUROPEAN PATENT SPECIFICATION

(11) **EP 2 170 233 B1**
(45) Date of publication and mention of the grant of the patent: **07.03.2018**
(21) Application number: 08776014.6
(22) Date of filing: 22.07.2008
(51) Int. Cl.: A61K 31/5375, A61K 9/70, A61F 13/00, A61P 17/02

(54) **METHODS AND PRODUCTS FOR IMPROVING WOUND HEALING**
VERFAHREN UND PRODUKTE FÜR VERBESSERTE WUNDHEILUNG
PROCÉDÉS ET PRODUITS POUR AMÉLIORER LA CICATRISATION

(30) Priority: 23.07.2007 GB 0714338
(43) Date of publication of application: 07.04.2010
(73) Proprietor: Sinclair Pharmaceuticals Limited, Godalming Surrey GU7 1XW (GB)
(72) Inventor: SJODIN, Olof, Torgny, Godalming Surrey GU7 1XW (GB)
(74) Representative: Gill Jennings & Every LLP
(86) International application number: PCT/GB2008/002493
(87) International publication number: WO 2009/013475

(56) References cited:
- WO-A-02/02061
- WO-A-2006/082393
- WO-A-2007/010294
- WO-A-2007/060413
- WO-A-2008/139170
- US-A1- 2008 064 711
- DATABASE REGISTRY Chemical Library, Supplier: UkrOrgSynthesis 20 January 2009 (2009-01-20), XP002511572 retrieved from STN accession no. RN1094544-90-7
- DATABASE REGISTRY Chemical Catalog, Supplier: Aurora Fine Chemical 6 May 2008 (2008-05-06), XP002511573 retrieved from STN accession no. RN1019574-04-9
- DATABASE REGISTRY Chemical Library, Supplier: Ambinter 15 November 2007 (2007-11-15), XP002511574 retrieved from STN accession no. RN953748-38-4

## Description

### Field of the Invention

This invention relates to methods and products useful in the treatment of wounds, in particular reducing the time required for a wound to heal.

### Background to the Invention

A wound is a physical trauma of the skin or mucosa. Wounds are commonly classified into "open" wounds such as incisions, lacerations, abrasions, punctures, penetrations and gun shot wounds, and "closed" wounds such as contusions and haematomas.

The healing of a wound is a complex, carefully regulated physiological response to a traumatic injury. Despite modern medical techniques, there remains a strong need for methods and compositions that improve wound healing, in particular reducing the time taken for a wound to heal.

### Summary of the Invention

The present invention is based on the surprising realisation that delmopinol, and its derivatives, is useful in healing a wound.

According to a first aspect of the invention, a morpholino compound of formula (I) 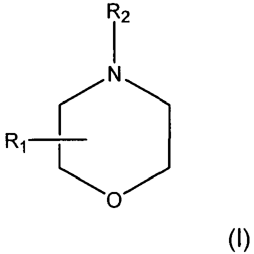 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof, is used in the manufacture of a medicament for promoting the healing of a wound.

According to a second aspect of the invention, a method of treating a wound comprises contacting a wound with a compound of formula (I) 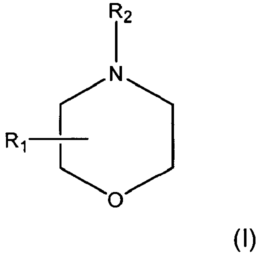 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof.

According to a third aspect of the invention, a material for contacting a wound is coated in or impregnated with a compound of formula (I) 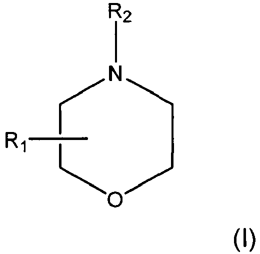 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof.

According to a fourth aspect of the invention, clothing is coated in or impregnated with a compound of formula (I) 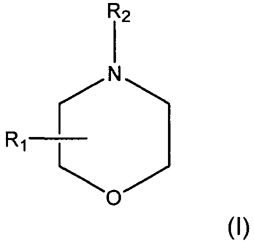 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof.

According to a fifth aspect of the invention, a compound of formula (I) 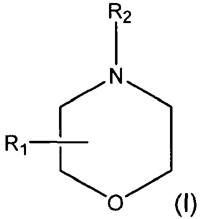 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof, is used in the manufacture of a medicament to prevent or reduce scarring of a wound, as it heals.

### Detailed Description of the Invention

The present invention is based on the surprising realisation that a compound of formula (I) can be used to improve the healing of a wound.

As used herein, the term "wound" is to be given its usual meaning in the art. According to the present invention, the wound is an "open" wound, wherein the skin or mucosa is broken, torn, burnt, cut or punctured. Preferred types of open wound would include abrasions, burns and scalds, incisions, lacerations, penetrations, puncture and gunshot wounds.

The claimed morpholino compounds are known *per se* as disclosed in US 4,894,221 and US 5,082,653.

In the present invention, the morpholino compounds are defined according to the general formula (I) shown above. In a preferred embodiment of the present invention, the sum of carbon atoms in the groups R₁ and R₂ is at least 10, and is preferably between 10 and 20. In a further preferred embodiment, the R₂ group terminates with the hydroxy group.

The preferred morpholino compound for use in the present invention is 3-(4-propyl-heptyl)-4-(2-hydoxyethyl) morpholine which is commonly known as Delmopinol (CAS No. 79874-76-3).

The morpholino compounds of the present invention can be used in their free base form or as a pharmaceutically acceptable salt thereof. Some examples of pharmaceutically acceptable salts are the salts of acids such as acetic acid, phosphoric, acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid and the like. The most preferred salt form is of hydrochloric acid. A preferred compound is delmopinol hydrochloride (CAS No. 98092-92-3).

The claimed compounds can be manufactured by any known method, for example, that disclosed in US 5,082,653 and WO 90/14342.

A claimed morpholino compound is preferably used in combination with an antimicrobial agent. The antimicrobial agent can be any pharmaceutically acceptable compound which is effective against pathogenic, preferably infectious, microbes. An antibiotic may therefore be used in a composition comprising a claimed morpholino compound. Any antibiotic that is effective against the pathogenic microbes may be used. Preferably, antibiotics are selected from the group consisting of clindamycin, erythromycin, benzylpencillin, tetracycline, chloramphenicol, vancomycin and linezolid.

The claimed compounds may, preferably and advantageously, be used in combination with a treatment that reduces the number of bacteria in a wound, i.e. an anti-bacterial agent, thereby reducing the chance of bacterial infection.

An anti-inflammatory agent may be used in a composition comprising a claimed morpholino compound. The skilled person will realise that the use of an anti-inflammatory agent is particularly useful in the treatment of inflammatory acne. Steroidal anti-inflammatories such as cortisone, or non-steroidal anti-inflammatories (NSAIDS), such as aspirin and ibuprofen, that inhibit cyclooxygenase isoenzymes are within the scope of the invention.

For the avoidance of doubt, compositions comprising all combinations of each of the ingredients described herein are within the scope of the invention.

Compositions for use in the present invention are preferably suitable for topical application to the mucosa or skin. In one embodiment, the composition consists of the claimed compound in a pharmaceutically acceptable cream, salve, ointment or gel. Compositions of the invention can be hydrophillic or hydrophobic. The composition can be an aqueous composition, although other suitable solvents, such as alcohols or other organic solvents, may be used. A combination of solvents may also be used.

A suitable cream may be prepared by incorporating the active compound in a topical vehicle such as light liquid paraffin, dispersed in a aqueous medium using surfactants. An ointment may be prepared by mixing the active compound with a topical vehicle such as a mineral oil or wax. A gel may be prepared by mixing the active compound with a topical vehicle comprising a gelling agent.

Topically administrable compositions may also comprise a matrix in which a pharmaceutically active compound of the present invention is dispersed so that the compound is held in contact with the skin in order to administer the compounds transdermally.

The claimed morpholino compound should be included in the composition at a level at which it is effective which can be determined on the basis of the severity of the condition. The compound of the invention may be present in any suitable concentration. Typically, the compound is present in a concentration of from 0.01% (w/v) to 20% (w/v) e.g. 15% (w/v), preferably from 0.01% (w/v) to 10% (w/v), preferably from 0.1% (w/v) to 5% (w/v) and most preferably from 0.1% (w/v) to 0.3% (w/v) e.g. 0.2% (w/v). A suitable level can easily be selected by a person skilled in the art.

Where an antimicrobial, antibiotic, anti-inflammatory agent and/or other additional component is included in the composition, these are also included at an effective level which can easily be determined by a person skilled in the art.

It has been found by the present inventor that, surprisingly, contacting a wound with a compound of formula (I) reduces the time required for the wound to heal. As used herein, "healing" of a wound refers to the physiological process wherein the wounded (damaged) area returns to an effectively normal state. When the wound is an open wound, healing refers to the process wherein the skin or mucosa re-forms a continuous barrier. The skilled person will realise that, after healing, the area of the wound may comprise scar tissue that is not identical to the surrounding tissue. The use of a compound of formula (I) can prevent or reduce scarring associated with wound healing. Contacting a wound with a compound of formula (I) can therefore prevent or reduce scarring, or reduce the unsightly appearance of scar tissue, compared to how the scar would form without contacting the wound with a compound of formula (I). A compound of formula (I) for use in a method of treating a wound comprises contacting the wound with a compound of formula (I). An example of treating a wound would be contacting the wound with a composition containing a compound of formula (I). Alternatively, the wound can be contacted with a material that is coated in or impregnated with a compound of formula (I). Preferably, the contact between the wound and the material comprising a compound of formula (I) is non-transient, i.e. there is a substantial period of contact. For example, contact can last for ten minutes or more, an hour or more, preferably 2, 3, 4 hours or more, such as 24 hours. The material comprising a compound of formula (I) can be woven (such as a fabric) or non-woven. Preferred materials include wound dressings such as a bandage, gauze, plaster or other medical dressings. Other fabrics intended for use in a medical environment, for example materials used in medical procedures or used in a hospital or other medical environment, such as sheets, pillowcases, linen and towels are preferred materials that can comprise a compound of formula (I).

According to a further aspect of the invention, clothing can comprise a compound of Formula (I). Preferably, the clothing is coated in or impregnated with a compound of formula (I). Clothing worn in a medical, preferably surgical, environment is a preferred embodiment. Examples of such medical clothing include the uniforms and clothing worn by medical practitioners such as doctors, in particular surgeons, and nurses, and the clothing worn by a patient such as a hospital gown. Clothing that may be worn when the chance of a wound occurring is high is preferred. Preferred clothing is therefore a gown worn during surgery or military clothing such as combat gear. In this embodiment, any wound that occurs while wearing the clothing will be contacted with a compound of formula (I) by means of the clothes surrounding and contacting the wound.

## Claims

1. A compound of formula (I) 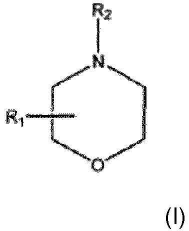 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof, for use in promoting the healing of an open skin wound.

2. A compound for use according to claim 1, wherein the open wound is an abrasion, incision, laceration, penetration, puncture or gunshot wound.

3. A compound for use according to any preceding claim, wherein the compound is coated or impregnated in a wound dressing.

4. A compound for use according to claim 3, wherein the wound dressing is a bandage, gauze, plaster or other medical dressing.

5. A compound for use according to any preceding claim, which is 3-(4-propyl-heptyl)-4-(2-hydroxyethyl) morpholine.

## Patentansprüche

1. Verbindung der Formel (I) 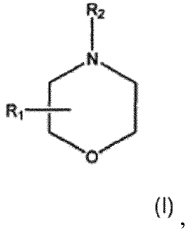 wobei R₁ eine gerade oder verzweigte Alkylgruppe, die 8 bis 16 Kohlenstoffatome enthält, an der 2- oder 3-Position des Morpholinorings ist, und R₂ eine gerade oder verzweigte Alkylgruppe, die 2 bis 10 Kohlenstoffatome enthält, ist, die mit einer Hydroxygruppe, außer in der Alphaposition, substituiert ist, oder pharmazeutisch verträgliche Salze davon für die Verwendung bei der Begünstigung der Heilung einer offenen Hautwunde.

2. Verbindung für die Verwendung nach Anspruch 1, wobei die offene Wunde eine Abschürfungs-, Schnitt-, Riss-, Stich-, Einstich- oder Schusswunde ist.

3. Verbindung für die Verwendung nach einem vorstehenden Anspruch, wobei die Verbindung auf einen Wundverband beschichtet oder imprägniert ist.

4. Verbindung für die Verwendung nach Anspruch 3, wobei der Wundverband eine Bandage, Gaze, ein Pflaster oder anderer medizinischer Verband ist.

5. Verbindung für die Verwendung nach einem vorstehenden Anspruch, die 3-(4-Propylheptyl)-4-(2-hydroxyethyl)morpholin ist.

## Revendications

1. Composé de formule (I) 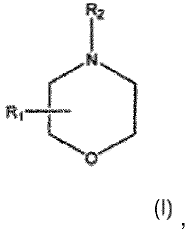 où R₁ représente un groupement alkyle linéaire ou ramifié contenant de 8 à 16 atomes de carbone en position 2 ou 3 du noyau morpholino et R₂ représente un groupement alkyle linéaire ou ramifié contenant de 2 à 10 atomes de carbone groupe substitué par un groupement hydroxy, sauf en position alpha, ou sels pharmaceutiquement acceptables de celui-ci, pour une utilisation permettant de favoriser la cicatrisation d'une plaie cutanée ouverte.

2. Composé pour une utilisation selon la revendication 1, où la plaie ouverte est une abrasion, une incision, une lacération, une plaie par pénétration, une plaie par arme blanche ou une plaie par balle.

3. Composé pour une utilisation selon l'une quelconque des revendications précédentes, où le composé est enduit ou imbibé dans un pansement.

4. Composé pour une utilisation selon la revendication 3, où le pansement est un bandage, une gaze, un plâtre ou tout autre pansement médical.

5. Composé pour une utilisation selon l'une quelconque des revendications précédentes qui est la 3-(4-propyl-heptyl)-4-(2-hydroxyéthyl)morpholine.
